# EUROPEAN PATENT APPLICATION

(11) **EP 2 312 305 A1**
(43) Date of publication of application: **20.04.2011**
(21) Application number: 11150664.8
(22) Date of filing: 05.05.2006
(51) Int. Cl.: G01N 27/403, G01N 33/00, G01N 33/497

(54) **Electrochemical sensor**

(30) Priority: 11.05.2005 GB 0509632
(62) Divisional of application: 06727030.6
(71) Applicant: Dart Sensors Limited, Exeter Devon EX4 4RN (GB)
(72) Inventor: King, Walter John, Exeter, Devon EX4 4RN (GB)
(74) Representative: Harrison, Ivor Stanley

(57) **Abstract**

An electrochemical sensor comprises noble metal electrodes in an electrolyte characterised in that the electrolyte comprises a monobasic acid selected from trifluromethanesulphonic acid, benzene sulphonic acid and perchloric acid.

## Description

This invention relates to improvements in and relating to electrochemical sensors and, in particular, provides an electrochemical sensor which can be operated at higher ambient temperatures compared with conventional electrochemical sensors and, thus, in a greater variety of applications.

Electrochemical sensors are used for detecting the presence of a particular "target" gas, usually but not necessarily a contaminating gas molecule, especially toxic gas molecules. However, electrochemical sensors may also be used in hygiene applications, for example to indicate the presence of an alcohol-containing disinfecting gel on the hands; in breath alcohol analysers; as air quality sensors operating in an enclosed environment such as a motor vehicle cabin; and for sensing the presence of carbon monoxide, especially to detect the presence of carbon monoxide in the pressurised air supply from a petrol-driven compressor, where carbon monoxide in the air supply would entail the risk of carbon monoxide poisoning. However, conventional sensors may not be capable of operating in the ambient conditions associated with the above applications.

Conventional electrochemical sensors are generally either of the two-electrode or the three-electrode type. Two-electrode sensors use fuel cell technology and comprise noble metal electrodes in an electrolyte and include a diffusion barrier through which the gas to be analysed passes before contact with the electrodes, reaction of the target gas (if present) with the electrodes causing a current to flow and, thus, providing an indication of the presence of the target gas. In a three-electrode potentiostatically controlled arrangement, the third electrode is generally referred to as a reference electrode, allowing the potential of the sensing electrode to be biased, thus controlling sensitivity to a particular target gas. The porous diffusion barrier, usually a PTFE membrane, acts as a support for the sensing electrode material and serves to maintain a dry region on the air side of the sensing electrode, to prevent contact with any free liquid electrolyte. The gas to be sensed for the presence of target gas molecules diffuses through the membrane into the electrocatalyst layer to reach active sites, being regions wetted with electrolyte where electrochemical reaction can occur to produce the output signal indicative of the presence of target gas molecules. However, the porous membrane is disadvantageous in that it retards the passage of the gas, thereby increasing the response time. This effect occurs even though membranes can have a porosity as high as 50% or even more. Furthermore, the membrane restricts the operating pressure of the sensor to atmospheric pressures or pressures approximating thereto; pressure variations above atmospheric pressure can damage or destroy the electrode structure and, thus, the sensor.

The response time is also affected by the absorption of the sensor electrolyte on the electrocatalysts. Since all electrolytes act, to some extent, as catalyst poisons, it is desirable where possible to select an electrolyte which has the least deleterious effect on the catalyst. However, the poisoning effect may be mitigated by the ambient conditions and, thus, phosphoric acid, which is a severe catalyst poison at room temperatures, can be used as an electrolyte at higher temperatures, where the poisoning effect is reduced.

Another factor which affects the response rate of electrochemical sensors is the internal resistance of the sensor, particularly in the case of the two-electrode sensors. Provided that the electrodes themselves are adequately conducting, the main source of internal resistance will be the electrolyte itself and, as most sensors contain an acid electrolyte, which is highly (ionically) conducting, the variable which will have the principal effect on resistance is the structure of the sensor. Some sensors have their electrodes in tandem, resulting in a long path length for conduction and thus a high internal resistance, this leading to a preference for a parallel-plate design in which the separation gap between the electrodes is as small as possible. Preferably, in order to minimise the possibility of contact resistances between components of the sensor, the sensor is manufactured as a single unit of the components (sensing electrode/separator plus electrolyte/counter electrode) rather than being separately assembled into the sensor housing. However, such a sensor would contain only a small amount of electrolyte and would thus be vulnerable to variations in temperature and humidity. Under conditions of high temperature and/or low humidity, water evaporates from the sensor and the electrolyte retreats from the electrode surfaces, thus altering its performance characteristics. On the other hand, under conditions of low temperature and/or high humidity, water is absorbed from the atmosphere resulting in a risk of flooding and possible leakage of acid from the sensor.

WO 00/77505 describes a carbon monoxide detector comprising pre-treatment means for removal of contaminating substances from a gaseous or vapour-phase atmosphere and conversion to non-contaminating substances and an electrochemical sensor comprising electrodes separated by an electrolyte absorbed on a porous substrate.

It is an object of the present invention to provide an electrochemical sensor which avoids the problems and disadvantageous of existing sensors.

In one aspect, the present invention provides an electrochemical sensor comprising sensing and counter electrodes and first (sensor) and second (reservoir) matrices each containing electrolyte, in which the first matrix is adjacent and in contact with the second matrix and has a catalytic coating applied thereto as sensing electrode, the matrices are of respectively different porosities with the resevoir matrix having the lowest surface area and the electrolyte contained within the second matrix is capable of flow to or from the first matrix.

In use, the ability of electrolyte to flow between the respective first and second matrices will maintain a substantially constant electrolyte volume in the first (sensor) matrix independent of ambient conditions of temperature and humidity.

Preferably, the catalytic coating applied to or disposed on the first matrix has the finest porosity or highest surface area, the sensor matrix itself having an intermediate porosity or surface area and the reservoir matrix having the lowest surface area. A higher surface area material will have a small pore size and, hence, a greater ability to retain fluid, compared with a lower surface area material of higher or coarser porosity. In sensors according to the invention, the counter electrode preferably comprises a catalytic coating applied to the other surface of the first matrix, although optionally the coating may be applied to a surface of the second matrix, adjacent the first.

The respective matrices, preferably in the form of disks formed from a plastics material or, for ambient temperature of approximately 120°C or higher, from a ceramics material, will generally be contained within a housing including means to admit a sample gas or a gas stream for test purposes. The test gas may be incident on the discs within the housing by passive diffusion or by an active or applied arrangement, such as by use of a diaphragm valve or a solenoid-driven piston. For a particular end-user application, for example as a hygiene sensor for determining the presence of a bactericide on the hands, the matrices may be configured to comply with the shape and dimensional requirements of the apparatus, such as a door handle, which is to carry the sensor. They may, therefore, be square or rectangular in particular embodiments.

Optionally, a third electrode may be provided; such third electrode may be disposed on the reservoir matrix remote from the centre matrix or on an additional matrix material.

The electrolyte for use in electrochemical sensors according to the invention is, for most purposes, sulphuric acid. Sulphuric acid has up to two ionisable hydrogen atoms and for most purposes is the industry standard electrolyte. It has been found that the poisoning effect of sulphuric acid on the catalyst varies with sulphuric acid concentration, whereby the catalyst is anodised and deactivated at sulphuric acid concentrations of 90% or higher, at which the incipient oxidising properties are stronger. As a practical measure, therefore, sulphuric acid would be suitable for use at slightly elevated ambient temperatures of, say, 35-50°C, where evaporation (and hence concentration) would not cause problems. However, it has been found that the poisoning effect by adsorption of the acid on the catalyst increases with increasing basicity of the acid, and so a monobasic acid such as trifluoromethanesulphonic acid, benzene sulphonic acid or perchloric acid may be preferred for ambient temperatures in the region of, say -10°C to 35°C, in order to enhance the electrode kinetics compared with sulphuric acid. At ambient temperatures in excess of say 50°C, the less anodising, tribasic, orthophosphoric acid (optionally blended with sulphuric or other acid) may be used, the improved kinetics of higher temperatures compensating for its higher intrinsic poisoning effect. Another consideration at higher temperatures, particularly temperatures in excess of 50°C, is that the matrix material becomes increasingly hydrophobic, whereby the presence of a wetting agent in the electrolyte is desirable. Some acids have intrinsic wetting properties and can thus be used on their own at higher temperatures even up to 90°C or above. Such wetting acids include monobasic acids or salts thereof, such as trifluoromethanesulphonic acid and benzene sulphonic acid. Trifluoromethanesulphonic acid, for example, can be used at a temperature of at least 75°C.

In one embodiment for use at higher ambient temperatures, the electrolyte comprises equal amounts by weight of phosphoric acid and sulphuric acid, the sulphuric acid being optional and intended to mitigate the poisoning effect of the phosphoric acid without causing anodisation, plus 5% sodium benzenesulphonate as wetting agent.

Electrochemical sensors according to the invention do not include a diffusion barrier and are able to operate under a greater range of ambient conditions than conventional sensors by virtue of the volume of electrolyte in the reservoir matrix being able to expand and contract to maintain a substantially constant volume in the sensor matrix. Electrochemical sensors according to the invention also have a short path length for diffusion of the test gas, an immobilised electrolyte and a parallel plate cell structure with unitary construction, thus minimising cell thickness.

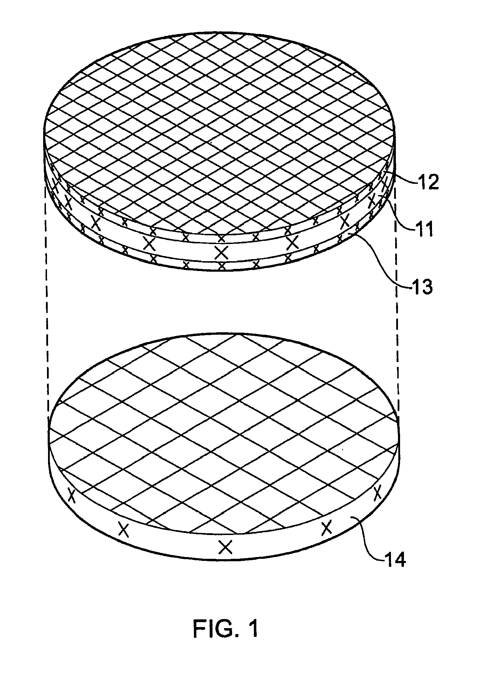

Embodiments of the invention will now be described by way of example with reference to the accompanying drawing, which is an exploded diagrammatic view of the component parts of an electrochemical sensor according to the invention for disposing in a suitable housing (not shown).

With reference to the drawing, a porous sensor disk (11) is formed from a plastics material, preferably polyvinyl chloride. The disk (11) is coated on each face with an electrocatalyst (12, 13) consisting of a metal of the platinum group, preferably platinum, as a dispersion having a high surface area, such a platinum black, preferably disposed on a support which may comprise a refractory ceramic oxide such as alumina, preferably itself having a high surface area, especially for use in ambient temperatures of 300°C or higher. In any event, the sensor disk should be formed from a ceramics material for use at ambient temperature above 100°C, preferably above 120°C. A reservoir matrix disk (14), also formed from a plastics material such as polyvinyl chloride subject to temperature requirements, is provided adjacent and in contact with the catalytic layer (13) disposed on the sensor disk; the porosity of the reservoir disk is higher than that of the sensor disk which itself has a higher porosity than the catalytic layers. The reservoir disk (14) contains absorbed electrolytic fluid, typically sulphuric acid and water.

In the components of the electrochemical sensor as illustrated, the catalytic layer (12) acts as the sensing electrode and the catalytic layer (13) acts as the counter electrode, the electrodes thus having a parallel plate structure. Since the catalytic electrode layers have the smallest pores, they have the strongest ability to retain fluid, whereas the sensor disk, having an intermediate porosity, will yield or take up electrolytes, as required, from the reservoir disk, thus maintaining a constant electrolyte volume within the catalytic layers. Variations in electrolyte volume are therefore accommodated by the reservoir disk, without the need for free liquid electrolyte, the sensor components remaining dry in appearance at all times.

Particular applications in which electrochemical sensors according to the invention may be used include the following:
hygiene sensors, in which is provided a real-time indication of the presence of an alcohol-containing disinfecting gel on the hands and which has the smallest practical diffusion path length from the hands to the sensor electrode and operates at ambient room temperatures, using a mono basic acid as electrolyte;
sensors in breath alcohol analysers operating at a temperature in the range of 35 to 40°C to avoid condensation from human breath and in which 4 M sulphuric acid is a preferred electrolyte, the raised temperature compensating for the inferior electro kinetics without adversely affecting the service life of the sensor;
air quality sensors for use in a motor vehicle cabin having the ability to withstand ambient temperatures up to 90°C and using, as electrolyte, a blend of phosphoric acid, sulphuric acid and benzene sulphonic acid (50:45:5% by weight) whereby the sensor withstands high temperatures encountered in hot conditions; and
carbon monoxide sensors operating at high pressures, for example where a petrol-powered compressor supplies pressurised air to an enclosed environment, where it is necessary to detect the presence of carbon monoxide (as a byproduct of petrol combustion) in the output air from the compressor.

In another aspect, therefore, the present invention provides a hygiene sensor, an alcohol sensor or an air quality sensor comprising an electrochemical sensor as hereinbefore described or using an electrolyte as hereinbefore described. In particular, the invention provides an alcohol sensor and an electrolyte therefor.

## Claims

1. An electrochemical sensor comprising noble metal electrodes in an electrolyte **characterised in that** the electrolyte comprises a monobasic acid selected from trifluromethanesulphonic acid, benzene sulphonic acid and perchloric acid.

2. An electrochemical sensor according to claim 1, in which the electrolyte includes a wetting agent.

3. An electrochemical sensor according to claim 2, in which the wetting agent comprises a monobasic acid or salt thereof and sulphuric and/or orthophosphoric acids.

4. Use of an electrochemical sensor according to any preceding claim as a hygiene sensor, an alcohol sensor or an air quality sensor.
